**Europäisches Patentamt**

**European Patent Office**

**Office européen des brevets**

(19)

(11) Veröffentlichungsnummer: **0 312 848**

**A1**

(12) # EUROPÄISCHE PATENTANMELDUNG

(21) Anmeldenummer: **88116589.8**

(22) Anmeldetag: **06.10.88**

(51) Int. Cl.⁴: **G10K 15/06 , G10K 11/30 , A61B 17/22**

(30) Priorität: **19.10.87 DE 3735345**

(43) Veröffentlichungstag der Anmeldung:
**26.04.89 Patentblatt 89/17**

(84) Benannte Vertragsstaaten:
**DE FR GB NL**

(71) Anmelder: **Siemens Aktiengesellschaft Berlin und München**
**Wittelsbacherplatz 2**
**D-8000 München 2(DE)**

(72) Erfinder: **Hassler, Dietrich, Dipl.-Ing.**
**Flurweg 3**
**D-8525 Uttenreuth(DE)**

(54) **Stosswellenquelle mit zentralem Ortungssystem.**

(57) Es wird von einem Lithotripter mit einer Stoßwellenquelle (2) zur Aussendung akustischer Impulse ausgegangen. Die Stoßwellenquelle (2) ist mit einer Fokussierungseinrichtung (24) versehen. Die von einer Abstrahlfläche (11) ausgesandten akustischen Impulse sind über ein Koppelmedium (36) auf ein zu zerstörendes Konkrement (K) lenkbar. Nach der Erfindung ist die Abstrahlfläche (11) in Abstrahlrichtung (12) nach außen gewölbt. Dabei ist die Fokussierungseinrichtung (24) so ausgebildet, daß die divergierende Wirkung dieser Abstrahlfläche (11) kompensiert ist. Weiterhin ist in der Stoßwellenquelle (2) ein zentraler stoßwellenfreier Raum (22) vorhanden.

FIG 1

EP 0 312 848 A1

## Stoßwellenquelle mit zentralem Ortungssystem

Die Erfindung betrifft einen Lithotripter mit einer Stoßwellenquelle zur Aussendung akustischer Impulse, die einen zentralen stoßwellenfreien Raum zum Einbringen eines Ortungssystems aufweist und die mit einer Fokussierungseinrichtung versehen ist, wobei die von einer Abstrahlfläche ausgesandten akustischen Impulse über ein Koppelmedium auf ein zu zerstörendes Konkrement lenkbar sind.

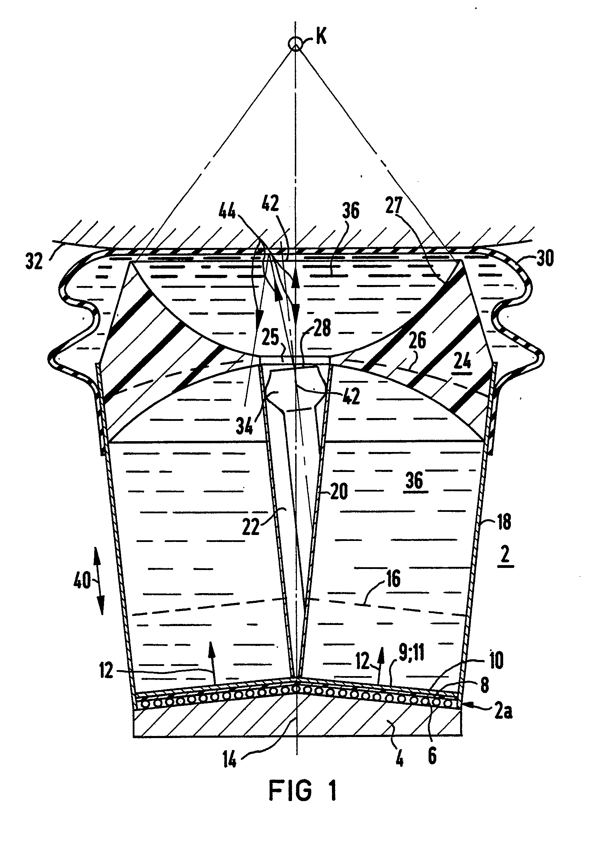

Ein Lithotripter der eingangs genannten Art ist in der EU-OS 0 148 653 in FIG 1 angegeben. Dort ist auf der Innenseite einer Kugelkalotte eine Vielzahl, z.B. 300-400, piezoelektrischer Einzelelemente mosaikförmig angeordnet. In der Mitte der Kalotte ist eine Aussparung angebracht, in der sich der Schallkopf einer Ultraschall-Ortungseinrichtung mit Bilddarstellungsgerät befindet. Als nachteilig ist hier anzusehen, daß der Schallkopf der Ultraschall-Ortungseinrichtung relativ weit von dem zu beobachtenden Konkrement entfernt ist. Das Konkrement selbst nimmt nur eine geringe Fläche in der Gesamtabbildung ein.

In der älteren deutschen Patentanmeldung P 37 27 691.3 (= VPA 87 P 3296 DE) wird vorgeschlagen, eine zentrale Öffnung in einer Stoßwellenquelle nach dem elektromagnetischen Prinzip vorzusehen, in die der Ultraschallkopf einer Ultraschallsende-und -empfangseinrichtung eingebracht wird. Die zentrale Öffnung in der Stoßwellenquelle benötigt für die Membran an ihrer Innenseite eine zusätzliche Einspannung.

Aufgabe der Erfindung ist es, bei einem Lithotripter der eingangs genannten Art einen zentralen stoßwellenfreien Raum in der Stoßwellenquelle zu schaffen, ohne daß an der Stoßwellenquelle selbst zusätzliche Einspannungen und Halterungen für die Membran notwendig sind; d.h., der konstruktive Aufwand zur Bereitstellung des stoßwellenfreien Raumes soll gering sein.

Die genannte Aufgabe wird erfindungsgemäß dadurch gelöst, daß die Abstrahlfläche in Abstrahlrichtung nach außen gewölbt ist, und daß die Fokussierungseinrichtung so ausgebildet ist, daß die divergierende Wirkung dieser Abstrahlfläche kompensiert ist.

Eine besonders einfache Konstruktion ergibt sich dann, wenn die Abstrahlfläche kegelmantelförmig ist.

Eine vorteilhafte Ausgestaltung zeichnet sich dadurch aus, daß die gewölbte Abstrahlfläche die Austrittsfläche einer akustisch divergierenden Linse ist. Dadurch ist es möglich, den aktiven Teil der Stoßwelle eben auszubilden.

Bei einer weiteren vorteilhaften Ausführungsform ist die Oberfläche des aktiven Teils der Stoßwellenquelle die gewölbte Abstrahlfläche. Dadurch sind die Verluste an akustischer Energie in der Stoßwellenquelle niedrig gehalten.

Bei allen Ausführungsbeispielen kann die Stoßwellenquelle vorteilhafterweise eine Stoßwellenquelle nach dem elektromagnetischen Prinzip sein.

Weitere Ausbildungen und Vorteile der Erfindung ergeben sich aus der nachfolgenden Beschreibung von Ausführungsbeispielen anhand zweier Figuren sowie aus den Unteransprüchen.

Es zeigen:

FIG 1 eine Stoßwellenquelle nach dem elektromagnetischen Prinzip im Längsschnitt, bei der die Oberfläche des aktiven Teils der Stoßwellenquelle als gewölbte Abstrahlfläche ausgebildet ist; und

FIG 2 einen Längsschnitt einer Stoßwellenquelle nach dem elektromagnetischen Prinzip, bei der die Austrittsfläche einer divergierenden akustischen Linse als gewölbte Abstrahlfläche ausgebildet ist.

Die in FIG 1 gezeigte Stoßwellenquelle 2 arbeitet nach dem elektromagnetischen Prinzip. Der aktive Teil 2a der Stoßwellenquelle 2 besteht bekannterweise aus einem schallharten runden Spulenträger 4 mit einer aufgeklebten Flachspule 6, einer sich daran anschließenden Isolierfolie 8 und einer randseitig befestigten metallischen Membran 10. Die Oberfläche der Membran 10 bildet die Oberfläche 9 des aktiven Teils 2a. Der Spulenträger 4 hat an der Trägerseite der Flachspule 6 Kegelform. Dieselbe Kegelform besitzen auch die Flachspule 6, die Isolierfolie 8 und die metallische Membran 10. Die Oberfläche 9 ist gleichzeitig die Abstrahlfläche 11 der Stoßwellenquelle 2. Sie ist somit in Abstrahl- oder Ausbreitungsrichtung der ausgesandten akustischen Impulse, verdeutlicht durch die beiden Pfeile 12, nach außen gewölbt. Dadurch ist die Ausbreitungsrichtung der akustischen Impulse nicht parallel zur Zentralachse 14 der Stoßwellenquelle 2; die Normalen und damit die Abstrahlrichtungen 12 bilden jeweils einen von Null verschiedenen Winkel bezüglich der Zentralachse 14.

Die emittierten Ultraschallimpulse divergieren. Die divergierende Ausbreitung der akustischen Impulse wird auch durch die gestrichelte Linie 16 veranschaulicht, die eine Stoßwellenfront darstellen soll.

Der aktive Teil 2a der Stoßwellenquelle 2, bestehend aus dem Spulenträger 4, der Flachspule 6, der Isolierfolie 8 und der metallischen Membran 10, ist in der kleineren Öffnung eines sich stetig erweiternden konischen Rohres oder Gehäuses 18 flüssigkeitsdicht befestigt. Die Wandung des Gehäuses 18 steht senkrecht auf der schrägen Membran 10, d.h. sie liegt in jedem Querschnitt parallel zu den Abstrahlrichtungen 12. Ebenfalls parallel zur Abstrahlrichtung 12 und zum Gehäuse 18 ist symmetrisch zur Zentralachse 14 eine Innenwandung 20 vorhanden. Sie grenzt einen zentralen stoßwellenfreien Raum 22 ab und hat die Form eines Kegelmantels. Der Kegel öffnet sich nach außen.

An der anderen, größeren Öffnung des Gehäuses 18, schließt sich eine Fokussierungseinrichtung 24 an. Die Fokussierungseinrichtung 24 ist hier eine bikonkave akustische Sammellinse. Sie weist im Bereich des stoßwellenfreien Raumes 22 eine zentrale Öffnung 25 auf. Sie muß (gegenüber einer zur Zentralachse 14 parallelen Stoßwellenausbreitung) die divergierende Wirkung der Abstrahlfläche 11 kompensieren. Die gestrichelte Linie 26 in der Fokussierungseinrichtung 24 deutet die (dünnere) Form der bikonkaven Sammellinse an, die diese bei (nicht gezeigter) achsparalleler Ausbreitungsrichtung der akustischen Impulse haben müßte, wenn die Außenbegrenzung 27 beibehalten wird. Die Kompensierung der divergierenden Wirkung der Abstrahlfläche 11 bedeutet also eine gewisse Verdickung der Sammellinse. Zu erwähnen ist noch, daß zur Vermeidung von sphärischen Aberrationen die Kontur der Sammellinse nach dem Snelliusschen Gesetz ausgebildet ist.

Am Gehäuse 18 ist außen in Höhe der Fokussierungseinrichtung 24 ein Ankoppelsack 30 flüssigkeitsdicht befestigt. Der flexible Ankoppelsack 30 dient der verlustarmen Ankopplung der Stoßwellenquelle 2 an die Hautoberfläche 32 eines Patienten während der Einwirkung auf das Konkrement K.

Im zentralen stoßwellenfreien Raum 22 ist zur Ortung und Beobachtung des Konkrementes K ein Ultraschallkopf oder Ortungssystem 34 einer Ultraschall-Ortungseinrichtung eingebracht. Der Schallkopf 34 bildet an der endseitigen Öffnung der konischen Innenwandung 20 einen flüssigkeitsdichten Abschluß. Der Innenraum der Stoßwellenquelle 2, der begrenzt ist von der Membran 10, dem Gehäuse 18, der Innenwandung 20, dem Ankoppelsack 30 und einem Teil des Schallkopfes 34, ist mit einem Koppelmedium oder einer Koppelflüssigkeit 36, bevorzugt mit entgastem Wasser, gefüllt. Der Druckausgleich zwischen den beiden Ankoppelräumen erfolgt über die Öffnung 25.

Der Fokus der Fokussierungseinrichtung 24 ist unveränderbar (Fixfokus). Er ist auf unterschiedlich tief liegende Konkremente K im Patienten ausrichtbar, indem der Abstand der gesamten Stoßwellenquelle 2 zur Hautoberfläche 32 änderbar ist. Das ist durch den Doppelpfeil 40 angedeutet. Dabei bleibt der innige Kontakt zwischen dem Ankoppelsack 30 und der Hautoberfläche bestehen. Das ist durch die Ausbildung des Faltenbalges am Ankoppelsack 30 gesichert. Weitere konstruktive Einzelheiten der Einstellvorrichtung, wie z.B. Halterungen und Ausgleichsgefäße für die Koppelflüssigkeit 36, sind hier nicht dargestellt.

Zur Vermeidung von Mehrfachreflexionen zwischen der Ultraschall-Austrittsfläche am Schallkopf 34 des Ortungssystems und der Ankoppelstelle am Patienten ist die Ultraschall-Austrittsfläche des Schallkopfes 34 schräg zur Normalen auf der Zentralachse 14 ausgerichtet. Die Flächennormale 42 der Ultraschall-Austrittsfläche bildet also einen von Null verschiedenen Winkel zur Zentralachse 14. Der Weg eines reflektierten Ultraschallstrahles ist durch die Pfeile 44 verdeutlicht. Aus der Wegdarstellung ist ersichtlich, daß nur die erste Reflexion, die die Lage der Hautoberfläche 32 angibt, auf den Schallkopf 34 wieder auftrifft. Weitere Reflexionen laufen am Schallkopf 34 vorbei.

Der Schallkopf 34 ist in diesem Ausführungsbeispiel entweder ein elektronischer Sektorscanner (Phased Array) oder ein mechanischer oder elektronischer Parallelscanner.

Für die Fokussierungseinrichtung 24 kommen Materialien in Frage, in denen die Schallgeschwindigkeit größer ist als die Schallgeschwindigkeit in Wasser. Es können aber auch fokussierende akustische Linsen verwendet werden, in denen die Schallgeschwindigkeit kleiner ist als in Wasser. Dann ist die äußere Formgebung (Kontur) der Fokussierungseinrichtung 24 jedoch konvex.

Die in FIG 1 beschriebene Ausführungsform hat den Vorteil, daß zur Erzeugung eines stoßwellenfreien Raumes 22 keine großen konstruktiven Änderungen am aktiven Teil 2a der Stoßwellenquelle 2 notwendig sind. Es genügt, dem aktiven Teil 2a außen eine kegelförmige Gestalt zu geben. Dadurch werden divergierende akustische Impulse ohne zusätzliche Verluste erzeugt, und zwar bei begrenztem Aufwand.

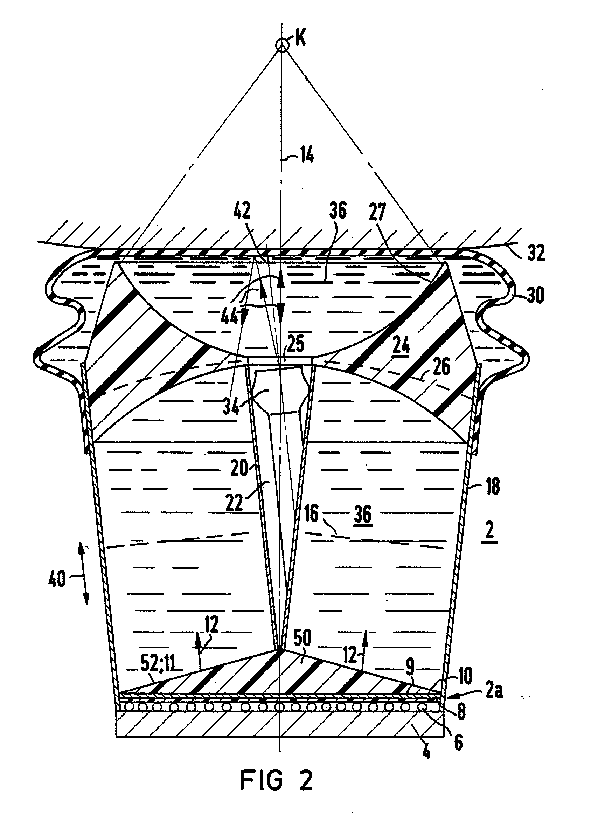

Das in FIG 2 gezeigte Ausführungsbeispiel ist ähnlich aufgebaut wie in FIG 1. Deshalb werden für gleiche Bauteile dieselben Bezugszeichen verwendet. Die Ausführungsform nach FIG 2 unterscheidet sich im wesentlichen nur im aktiven Teil 2a der Stoßwellenquelle 2. Der Spulenträger 4 ist hier eine kreisförmige Scheibe mit konstanter Dicke. Auf dem Spulenträger 4 sind ebenfalls die Flachspule 6 sowie die Isolierfolie 8 und die Membran 10 angeordnet. Jedoch sind diese Bauteile im Gegensatz zu FIG 1 eben. Die Ausbreitungsrichtung der von der Membran 10 erzeugten akustischen Impulse liegt somit parallel zur Zentralachse 14. Im Gegensatz zu FIG 1 ist an die Membran 10 eine divergierende akustische Linse 50 akustisch gut angekoppelt. Die Außen- oder Austrittsfläche 52 der Linse 50 ist hier die Abstrahlfläche 11. Sie ist in Abstrahlrichtung 12 nach außen gewölbt. Die Wölbung ist kegelmantelförmig. Jede Abstrahlrichtung 12 steht in einem bestimmten Winkel auf der Abstrahlfläche 11, der sich nach dem Berechnungsgesetz aus dem Verhältnis der Schallgeschwindigkeiten der angrenzenden Medien ergibt. D.h., hier steht die Abstrahlfläche 12 nicht senkrecht auf der Abstrahlfläche 11.

Der weitere Aufbau entspricht dem in FIG 1 mit der Ausnahme, daß die Spitze der Innenwandung 20 hier die Spitze der akustischen Linse 50 berührt, nicht die Spitze der Membran 10 (wie in FIG 1).

Der Vorteil der in FIG 2 gezeigten Ausführungsform besteht darin, daß der aktive Teil 2a der Stoßwellenquelle 2 in bekannter Weise aufgebaut sein kann. Durch akustische Ankopplung der Linse 50 an die Membran 10 ist ein zentraler stoßwellenfreier Raum 22 geschaffen, in den der Schallkopf 34 einer Ultraschall-Ortungseinrichtung einbringbar ist. Die gegenüber FIG 1 zusätzlichen Verluste an akustischer Energie in der Linse 50 können ohne weiteres in Kauf genommen werden.

Die in FIG 1 und FIG 2 gezeigten Ausführungsbeispiele besitzen durch einfache konstruktive Maßnahmen einen zentralen schallfreien Raum 22, in den der Schallkopf 34 einer Ultraschall-Ortungsvorrichtung leicht einbringbar ist. Die Membran 10 muß in beiden Fällen nicht durch besonders aufwendige Halteeinrichtungen gestützt werden.

**Ansprüche**

1. Lithotripter mit einer Stoßwellenquelle zur Aussendung akustischer Impulse, die einen zentralen stoßwellenfreien Raum zum Einbringen eines Ortungssystems aufweist und die mit einer Fokussierungseinrichtung versehen ist, wobei die von einer Abstrahlfläche ausgesandten akustischen Impulse über ein Koppelmedium auf ein zu zerstörendes Konkrement lenkbar sind,
**dadurch gekennzeichnet,**
daß die Abstrahlfläche (11) in Abstrahlrichtung (12) nach außen gewölbt ist, und daß die Fokussierungseinrichtung (24) so ausgebildet ist, daß die divergierende Wirkung dieser Abstrahlfläche (11) kompensiert ist.

2. Stoßwellenquelle nach Anspruch 1,
**dadurch gekennzeichnet,**
daß die Abstrahlfläche (11) kegelmantelförmig ist (FIG 1 und 2).

3. Stoßwellenquelle nach Anspruch 1 oder 2,
**dadurch gekennzeichnet,**
daß die Oberfläche (9) des aktiven Teils (2a) der Stoßwellenquelle (2) die gewölbte Abstrahlfläche (11) ist (FIG 1).

4. Stoßwellenquelle nach Anspruch 1 oder 2,
**dadurch gekennzeichnet,**
daß die Austrittsfläche (52) einer divergierenden akustischen Linse (50) die gewölbte Abstrahlfläche (11) ist (FIG 2).

5. Stoßwellenquelle nach einem der Ansprüche 1 bis 4,
**dadurch gekennzeichnet,**
daß die Stoßwellenquelle (2) eine Stoßwellenquelle nach dem elektromagnetischen Prinzip ist (FIG 1 und 2).

6. Stoßwellenquelle nach einem der Ansprüche 1 bis 5,
**dadurch gekennzeichnet,**
daß das Ortungssystem (34) ein Ultraschall-Ortungssystem ist (FIG 1 und 2).

7. Stoßwellenquelle nach einem der Ansprüche 1 bis 6,
**dadurch gekennzeichnet,**
daß das Ortungssystem (34) ein elektronisches Phased Array oder ein lineares Array ist.

8. Stoßwellenquelle nach einem der Ansprüche 1 bis 7,
**dadurch gekennzeichnet,**
daß die Fokussierungseinrichtung (24) im Bereich des stoßwellenfreien Raumes (22) eine zentrale Öffnung (25) aufweist.

9. Stoßwellenquelle nach einem der Ansprüche 1 bis 8,
**dadurch gekennzeichnet,**
daß die Fokussierungseinrichtung (24) eine bikonkave akustische Sammellinse ist.

10. Stoßwellenquelle nach einem der Ansprüche 1 bis 9,
**dadurch gekennzeichnet**
daß die gewölbte Abstrahlfläche (11) unmittelbar an das von einem Gehäuse (18) gehaltene Koppelmedium (36) anschließt, das mit der Fokussierungseinrichtung (24) in Berührung steht, die wiederum nach außen hin von einem mit Koppelmedium (36) gefüllten Ankoppelsack (30) eingefaßt ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-3 506 583 (SIEMENS)<br>* Zusammenfassung; Figur *<br>--- | 1,2,4-7,9 | G 10 K 15/06<br>G 10 K 11/30<br>A 61 B 17/22 |
| Y | US-A-4 001 766 (HURWITZ)<br>* Insgesamt *<br>--- | 1,2,4-7,9 | |
| Y | FR-A-2 591 467 (WOLF)<br>* Zusammenfassung; Seite 6, Zeile 33 - Seite 7, Zeile 7; Seite 9, Zeile 16 - Seite 10, Zeile 5 *<br>----- | 1,2,4-7,9 | |

| | RECHERCHIERTE SACHGEBIETE (Int. Cl.4) |
|---|---|
| | G 10 K<br>G 01 S<br>A 61 B |

Der vorliegende Recherchenbericht wurde für alle Patentansprüche erstellt

| Recherchenort | Abschlußdatum der Recherche | Prüfer |
|---|---|---|
| DEN HAAG | 12-12-1988 | OLDROYD D.L. |